# FASCICULE DE BREVET EUROPEEN

(11) **EP 4 221 664 B1**
(45) Date de publication et mention de la délivrance du brevet: **23.07.2025**
(21) Numéro de dépôt: 21782763.3
(22) Date de dépôt: 30.09.2021
(51) Int. Cl.: A61K 8/02, A61K 8/04, A61K 8/46, A61K 8/73, A61Q 5/02, A61Q 19/10

(54) **NOUVELLE COMPOSITION COSMÉTIQUE SOUS FORME DE GRANULÉS**
NEUE KOSMETISCHE ZUSAMMENSETZUNG IN GRANULATFORM
NOVEL COSMETIC COMPOSITION IN THE FORM OF GRANULES.

(30) Priorité: 01.10.2020 FR 2010045
(43) Date de publication de la demande: 09.08.2023
(73) Titulaire: Pierre Fabre Dermo-Cosmétique, 81500 Lavaur (FR)
(72) Inventeur: LEMERCIER, Laetitia, 31130 FLOURENS (FR); TRANNOY, Philippe, 31400 TOULOUSE (FR)
(74) Mandataire: Ipside
(86) Numéro de dépôt international: PCT/EP2021/077019
(87) Numéro de publication internationale: WO 2022/069671

(56) Documents cités:
- WO-A2-2009/153311
- CN-A- 105 687 054
- FR-A1- 3 030 269
- FR-A1- 3 068 243
- JP-A- H06 271 417
- US-A- 6 063 366

## Description

La présente invention concerne le domaine du soin et de l'hygiène de la peau et/ou des fibres kératiniques, en particulier humaines, notamment des cheveux.

Plus particulièrement, la présente invention concerne une composition cosmétique sous forme sèche du type convenant à une application topique sur la peau et/ou les fibres kératiniques, notamment humaines, ainsi qu'un granulé délitable convenant à une telle application et un procédé de préparation d'un tel granulé délitable. L'invention concerne également un procédé de lavage et/ou de traitement cosmétique des fibres kératiniques, notamment humaines, ainsi qu'un procédé de lavage et/ou de traitement cosmétique de la peau, notamment humaine, ces deux procédés mettant en œuvre une composition cosmétique selon l'invention. La présente invention concerne également l'utilisation cosmétique d'une telle composition pour le gommage de la peau du visage et/ou du corps d'un individu.

### ETAT DE LA TECHNIQUE

Dans le domaine du lavage des fibres kératiniques, en particulier des poils et des cheveux, de nombreux produits cosmétiques de lavage sont connus. Ces produits sont généralement destinés à nettoyer les fibres kératiniques tout en leur apportant de bonnes propriétés cosmétiques. Les produits classiques de nettoyage des fibres kératiniques, tels que les gels douches et les shampoings, se présentent le plus souvent sous forme de liquides plus ou moins épaissis. Mais ces produits peuvent se révéler difficiles à doser : plus ils sont liquides, plus ils ont tendance à couler entre les doigts, rendant leur dosage difficile et générant du gaspillage, et plus ils peuvent fuir hors de leur conditionnement. Pour ces raisons, il peut être avantageux d'utiliser des produits moussants sous forme sèche, qui ne contiennent pas ou très peu d'eau.

Les shampoings secs existent depuis de nombreuses années, soit sous forme poudreuse, soit sous forme d'aérosols. Ils permettent d'éliminer l'excès de sébum d'une façon rapide sans mouiller la chevelure. Ils agissent en captant le sébum par absorption grâce à des poudres choisies pour leurs qualités absorbantes de sébum. Les poudres employées peuvent être d'origine minérale, organique ou synthétique, et peuvent notamment être des dérivés d'amidon de blé, de riz et de maïs. Dans la pratique, les shampoings secs proposés dans le commerce ne donnent pas entière satisfaction. En particulier, la sensation de propreté perçue par les utilisateurs n'est pas persistante tout au long de la journée, notamment après des activités physiques. Par ailleurs, leurs propriétés coiffantes, telles qu'apport de volume à la chevelure et décollement des racines, sont en général très limitées. En outre, ils ne permettent pas d'éliminer efficacement l'excès de sébum présent sur le cuir chevelu et les désagréments occasionnés comme les irritations ou les odeurs.

De plus, la stabilité des shampoings sous forme d'aérosols comprenant des poudres est difficile à obtenir.

Les compositions cosmétiques sous forme de poudre sèche, qu'elles soient destinées à une application sur la peau ou sur les fibres kératiniques, ne permettent quant à elles pas d'obtenir un démarrage de mousse au moment souhaité, et elles ne permettent pas toujours d'obtenir une abondance de mousse suffisante. En outre, lors de l'utilisation de poudres pulvérulentes, les utilisateurs peuvent avoir du mal à les éliminer au rinçage et il peut en rester des résidus sur la peau ou les cheveux.

Enfin, les compositions sèches sous forme de poudres sont difficiles à manipuler, tant lors de leur fabrication que lors de leur utilisation. Un exemple de telle composition sèche sous forme de poudre est décrit dans le document CN105687054.

Il a été proposé par l'art antérieur des compositions cosmétiques solides sous forme de granulés, plus faciles à manipuler que les poudres. On peut citer à cet égard le document FR 3 030 269, qui décrit des compositions cosmétiques anhydres solides sous forme de particules comprenant au moins 30 % en poids de tensioactif(s) anionique(s), au moins 5 % en poids de tensioactif(s) amphotère(s) et au moins 10 % en poids de charge(s) ; ou encore le document FR 3 068 243 qui décrit des compositions cosmétiques solides sous forme de granulés contenant un tensioactif anionique choisi parmi les dérivés d'acide iséthionique, au moins un tensioactif anionique choisi parmi les dérivés d'acide glutamique, au moins un tensioactif amphotère et au moins 20 % en poids de charges. Ces compositions sous forme de granulés génèrent, au contact de l'eau, une mousse très abondante de façon très rapide, cette mousse n'étant en outre pas stable dans le temps. Elles présentent notamment l'inconvénient de nécessiter une quantité importante d'eau de rinçage.

Il existe donc un besoin de mettre au point de nouvelles compositions de soin et d'hygiène de la peau et/ou des fibres kératiniques, telles que les poils et les cheveux, qui soient faciles à manipuler, qui permettent de former au contact de l'eau de la mousse de manière maitrisée, sur le plan de la vitesse de formation de la mousse, pour assurer le meilleur confort d'utilisation possible à l'utilisateur, et sur le plan de la quantité de mousse formée, de sorte à limiter la quantité d'eau de rinçage requise, ceci tout en présentant une performance de lavage et/ou de soin cosmétique au moins aussi bonne, voire meilleure, que celle des compositions sèches proposées par l'art antérieur, c'est-à-dire de mettre au point des compositions cosmétiques offrant à la fois une activité nettoyante optimale avec un démarrage de mousse de vitesse optimale et une abondance de mousse optimale, et de bonnes propriétés cosmétiques, notamment, dans le cas du traitement cosmétique des cheveux, un apport de volume à la chevelure, avec une sensation de douceur et une sensation de propreté durable dans le temps. La présente invention vise à atteindre ces objectifs. Un objectif supplémentaire de l'invention est que la mousse formée lors de la mise en contact de la composition avec l'eau soit suffisamment stable durant toute la durée normale d'utilisation d'une telle composition par les consommateurs, afin d'optimiser l'efficacité nettoyante de la composition. Typiquement, pour un shampoing par exemple, cette durée est d'environ 5 minutes.

### RESUME DE L'INVENTION

De façon surprenante et inattendue, les inventeurs ont mis en évidence que des compositions cosmétiques lavantes sous forme sèche comprenant au moins 50 % en volume de granulés délitables, de constitution particulière, génèrent au contact de l'eau une quantité de mousse suffisante et suffisamment stable dans le temps pour assurer une bonne efficacité de lavage, sans nécessiter une quantité d'eau excessive pour le rinçage, le délitement des granulés étant en outre avantageusement progressif, si bien que la génération de mousse est légèrement retardée dans le temps et il est obtenu un effet exfoliant initial. Ces résultats avantageux sont associés à la présence dans les granulés délitables, outre au moins un tensioactif anionique et au moins un agent absorbant, d'un ou plusieurs agents épaississants choisis parmi les gommes, dans une quantité totale comprise entre 1 et 4 % en poids par rapport au poids total de la composition. De telles compositions apportent les propriétés nettoyantes attendues d'une composition cosmétique pour le lavage de la peau et/ou des fibres kératiniques, notamment d'un shampoing ou d'un masque, ainsi, dans ce dernier cas, que des propriétés coiffantes avantageuses telles qu'un apport de volume et de masse à la chevelure avec une sensation de douceur, de propreté et de fraicheur persistante dans le temps ; mais aussi des propriétés assainissantes du cuir chevelu. Ces nouvelles compositions cosmétiques sont également particulièrement intéressantes par leur facilité de manipulation lors de leur fabrication et de leur conditionnement, par leur facilité d'utilisation, notamment en ce qu'elles réduisent, par rapport à des compositions pulvérulentes classiques, la présence de poussières lors de leur utilisation. Elles sont en outre particulièrement avantageuses en ce qu'elles permettent, par le contrôle des caractéristiques des granulés, notamment leur taille, un contrôle plus fin encore de la vitesse de formation de mousse par contact du tensioactif anionique avec de l'eau, cette vitesse pouvant être facilement adaptée en fonction de l'application particulière visée.

Par ailleurs, ces compositions cosmétiques selon l'invention, par la présence des granulés et leur lente vitesse de délitement, sont dotées d'activité exfoliante du fait même des caractéristiques physiques de ces granulés, ce qui les rend particulièrement avantageuses pour les applications de nettoyage et de soin cosmétique de la peau et/ou des fibres kératiniques, notamment humaines.

Selon un premier aspect, l'invention concerne ainsi une composition cosmétique sous forme sèche, convenant à une application topique sur la peau et/ou les fibres kératiniques, comprenant au moins 50 % en volume de granulés délitables présentant une taille, mesurée par tamisage, comprise entre 150 et 2000 µm et contenant un mélange de :
- au moins un tensioactif anionique,
- au moins un agent absorbant choisi parmi les amidons,
- et au moins un agent épaississant choisi parmi les gommes d'origine végétale, microbienne et/ou synthétique,
la quantité totale dans la composition de ce ou ces agents épaississants choisis parmi les gommes d'origine végétale, microbienne et/ou synthétique étant comprise entre 1 et 4% en poids par rapport au poids total de la composition, de préférence comprise entre 2 et 4 % en poids par rapport au poids total de la composition.

Préférentiellement, la quantité totale de ce ou ces agents épaississants choisis parmi les gommes d'origine végétale, microbienne et/ou synthétique, présents dans la composition, est contenue dans les granulés délitables.

Selon un deuxième aspect, l'invention concerne un procédé de lavage et/ou de traitement cosmétique non thérapeutique des fibres kératiniques, comportant une étape d'application, sur les fibres kératiniques humides, d'une telle composition cosmétique sous forme sèche comprenant au moins 50 % en volume de granulés délitables, cette étape d'application étant suivie optionnellement d'un massage pour développer de la mousse, puis d'un temps de pose optionnel de 0 à 5 minutes, puis d'un rinçage à l'eau des fibres kératiniques.

Selon un troisième aspect, l'invention concerne un procédé de lavage et/ou de traitement cosmétique non thérapeutique de la peau, comportant une étape d'application, sur la peau humide, d'une telle composition cosmétique sous forme sèche comprenant au moins 50 % en volume de granulés délitables, cette étape d'application étant suivie optionnellement d'un massage pour développer de la mousse, puis d'un temps de pose optionnel de 0 à 5 minutes, puis d'un rinçage de la peau à l'eau.

Selon un quatrième aspect, l'invention concerne l'utilisation cosmétique non thérapeutique, pour le gommage de la peau du visage et/ou du corps d'un individu, d'une telle composition cosmétique sous forme sèche comprenant au moins 50 % en volume de granulés délitables.

Selon un cinquième aspect, l'invention concerne un granulé délitable convenant à une application topique sur la peau et/ou les cheveux d'un individu, susceptible d'entrer dans la constitution de la composition selon l'invention, telle que décrite ci-avant, ce granulé délitable présentant une taille, mesurée par tamisage, comprise entre 150 et 2000 µm et contenant un mélange de :
- au moins un tensioactif anionique,
- au moins un agent épaississant choisi parmi les gommes d'origine végétale, microbienne et/ou synthétique,
- et au moins un agent absorbant choisi parmi les amidons, la quantité totale dudit ou desdits agents épaississants choisis parmi les gommes d'origine végétale, microbienne et/ou synthétique, dans ce granulé, étant comprise entre 1 et 4 % en poids, de préférence comprise entre 2 et 4 % en poids, par rapport au poids total du granulé.

Un autre aspect décrit présentement concerne un procédé de préparation d'un tel granulé délitable, ce procédé comprenant des étapes successives de :
- préparation d'un mélange contenant ledit au moins un tensioactif anionique, ledit au moins un agent absorbant et ledit au moins un agent épaississant, en quantité comprise entre 1 et 4 % en poids, de préférence entre 2 et 4 % en poids, par rapport au poids total du granulé,
- ajout d'un liquide de mouillage à ce mélange,
- formation d'un granulé,
- et séchage de ce granulé.

### Définitions

Dans la présente invention, on entend par « granulé », de manière classique en elle-même, une préparation constituée par des grains solides et secs formés chacun par un agglomérat cohésif de particules de poudre, les granulés étant typiquement obtenus par un procédé de granulation.

Par « délitable », on entend une capacité d'une entité solide à se désagréger, à plus ou moins grande vitesse, en présence d'eau et/ou sous l'effet d'un frottement mécanique exercé sur elle.

Par « poudre », on entend au sens de la présente invention, de manière classique en elle-même, une forme galénique constituée de particules solides, sèches, libres, chacune étant formée d'un unique composant.

Par « forme sèche », on entend au sens de la présente invention, une composition cosmétique qui ne comprend pas d'eau / anhydre, ou une composition comprenant une teneur en eau inférieure ou égale à 5% en poids, de préférence inférieure ou égale à 2% en poids, de façon préférée inférieure ou égale à 1% en poids, et de façon encore préférée inférieure ou égale à 0,5% en poids, par rapport au poids total de la composition, ou une composition cosmétique qui ne contient pas d'eau ajoutée, c'est-à-dire que l'eau éventuellement présente dans la composition cosmétique selon l'invention n'est rien d'autre que de l'eau liée, comme de l'eau de cristallisation des sels, ou des traces d'eau absorbées par les matières premières utilisées dans la réalisation de la composition cosmétique, ou encore de l'eau résiduelle provenant d'un procédé de granulation par voie humide éventuellement mis en œuvre pour la formation de granulés.

Par « masque », on entend au sens de la présente invention, un type de soin du visage et/ou du cuir chevelu consistant en l'application prolongée d'un produit cosmétique sur le visage, le cuir chevelu et/ou les cheveux. Ce masque est un produit destiné à être appliqué sur peau/cheveu humide/mouillé puis à être rincé après un temps de pose.

Par « application topique », on entend au sens de la présente invention, une application sur la peau (dont le cuir chevelu), les muqueuses et/ou les fibres kératiniques.

Par « fibres kératiniques », on entend au sens de la présente invention, les cheveux, les cils, les sourcils et les poils, préférentiellement les poils de la barbe. Selon l'invention, la barbe inclut la moustache.

Par « cosmétiquement acceptable », on entend au sens de la présente invention, ce qui est utile dans la préparation d'une composition cosmétique, qui est généralement sûr, non toxique et ni biologiquement ni autrement non souhaitable, et qui est acceptable pour une utilisation cosmétique, notamment par application topique au niveau de la peau (dont le cuir chevelu), les muqueuses et les fibres kératiniques.

Par « tensioactif anionique », on entend au sens de la présente invention, un tensioactif ne comportant à titre de groupements ioniques ou ionisables que des groupements anioniques. Dans la présente invention, on qualifie une entité comme étant « anionique » lorsqu'elle possède au moins une charge négative permanente ou lorsqu'elle peut être ionisée en une entité chargée négativement, notamment dans les conditions d'utilisation de la composition de l'invention (milieu, pH par exemple), et qu'elle ne possède pas de charge cationique.

Par « agent absorbant », on entend un agent apte à absorber un fluide, par exemple le sébum.

Dans la présente description, et à moins d'une indication contraire, l'expression « au moins un » est équivalente à l'expression « un ou plusieurs » et peut y être substituée. L'expression « compris entre » est équivalente à l'expression « allant de... à... » et peut y être substituée, et sous-entend que les bornes sont incluses.

### DESCRIPTION DETAILLEE DE L'INVENTION

Selon un premier aspect, l'invention concerne une composition cosmétique sous forme sèche, plus particulièrement sous forme d'un solide divisé, convenant à une application topique sur la peau et/ou les fibres kératiniques, en particulier d'un individu humain. Cette composition cosmétique comprend au moins 50 % en volume, par rapport au volume total de la composition, de granulés délitables contenant un mélange d'au moins un tensioactif anionique, au moins un agent épaississant et au moins un agent absorbant. On entend par là que chaque granulé individuel contient, en mélange, au moins un tensioactif anionique, au moins un agent épaississant et au moins un agent absorbant.

La mise en œuvre de granulés, en quantité volumique majoritaire dans la composition, permet notamment, de manière particulièrement avantageuse, d'augmenter la capacité d'écoulement de la composition, ce qui facilite en particulier son extraction hors d'un sachet dans lequel elle est conditionnée. Elle facilite également la manipulation de la composition lors de sa fabrication et de son conditionnement, et lors de son utilisation, et réduit le dégagement de poussière par la composition. La forme solide de la composition facilite son stockage et son transport.

Ces avantages sont obtenus sans pour autant pénaliser l'efficacité lavante et de soin cosmétique de la composition, voire même en améliorant cette efficacité.

Tout d'abord, il est observé que la forme en granulés permet d'assurer un mélange homogène et uniforme des constituants de ces derniers, en particulier du tensioactif anionique, de l'agent épaississant et de l'agent absorbant, ce qui améliore les performances de lavage et de soin de la composition cosmétique selon l'invention. Ces performances sont particulièrement bonnes, tant en ce qui concerne le nettoyage de la zone visée, que la sensation de propreté perçue par l'utilisateur, et les effets cosmétiques obtenus, par exemple l'apport de volume à la chevelure dans le cas des shampoings ou des masques pour les cheveux. L'action des constituants particuliers des granulés que sont le tensioactif anionique, l'agent épaississant et l'agent absorbant, n'est ainsi seulement nullement empêchée, mais elle s'exerce au contraire de manière particulièrement importante.

Le tensioactif anionique assure notamment un effet lavant important, et la formation de mousse au contact de l'eau.

L'agent absorbant permet quant à lui avantageusement d' absorber tout fluide gras présent sur la peau ou les fibres kératiniques, comme les salissures lipophiles et notamment l'excès de sébum. Lors de la fabrication des granulés, il facilite en outre la granulation en jouant le rôle de liant. Il peut également permettre d'intégrer des substances fluides, qu'il est apte à absorber, à l'intérieur des granulés.

En outre, un réglage adéquat des caractéristiques physiques des granulés, notamment de leur taille et leur forme, en association avec la présence en leur sein d'un ou plusieurs agents épaississants choisis parmi les gommes d'origine végétale, microbienne et/ou synthétique, dans la plage de concentration spécifique préconisée par l'invention, permet de contrôler leur vitesse de délitement, en particulier en présence d'eau, et de ce fait la durée de leur effet exfoliant, et la vitesse de formation de mousse en présence d'eau.

Les granulés mis en œuvre dans la composition selon l'invention présentent avantageusement une vitesse de délitement plutôt lente. On entend par là que le granulé ne se désagrège pas trop rapidement, en présence d'eau et/ou lorsque des frottements mécaniques répétés sont exercés sur lui, par exemple par la main de l'utilisateur.

Le temps pour obtenir un délitement complet des granulés selon l'invention, mis en présence d'eau et/ou soumis à frottements, est de préférence supérieur à 30 secondes, de préférence supérieur à 1 minute, de façon préférée supérieur à 2 minutes, et préférentiellement encore supérieur à 3 minutes. Ce temps est en outre de préférence inférieur à 5 minutes.

Le temps de délitement d'un granulé selon l'invention peut par exemple être déterminé en mettant la composition selon l'invention dans l'eau, par exemple à raison de 3 g de composition dans 100 ml d'eau, à 25°C, sous agitation, par exemple à 200 tours/min, et en déterminant le temps nécessaire à la disparition complète des granulés contenus dans la composition.

Préférentiellement, les granulés mis en œuvre dans la composition selon l'invention sont tels que, lorsqu'ils sont soumis au protocole ci-dessus, il est observé la présence partielle de granulés après 3 minutes, et une absence de granulés après 4 minutes.

Les granulés mis en œuvre dans la composition selon l'invention peuvent aussi bien avoir été obtenus par un procédé de granulation en voie sèche que par un procédé de granulation humide.

Dans des modes de réalisation particuliers de l'invention, la composition cosmétique comprend au moins 75 % en volume, par rapport au volume total, desdits granulés.

La composition peut contenir, outre les granulés, tout autre ingrédient cosmétiquement acceptable, tel qu'une charge, un parfum, etc., chacun de ces ingrédients se présentant bien entendu sous forme d'un solide à l'état divisé ou étant absorbé sur un solide à l'état divisé.

Les granulés mis en œuvre dans la composition peuvent également contenir tout autre ingrédient cosmétiquement acceptable.

Les ingrédients contenus dans la composition selon l'invention sont de préférence d'origine naturelle, répondant ainsi à une demande très forte de formulations contenant des ingrédients d'origine naturelle marquant, depuis quelques années, le marché cosmétique. Les consommateurs désirent en effet des formulations exemptes de matières chimiques auxquels ils préfèrent des ingrédients d'origine naturelle, réputés pour leur meilleure tolérance et affinité avec la peau, et qui soient plus respectueuses de l'environnement.

Dans des modes de réalisation particuliers de l'invention, la composition cosmétique comprend un ou plusieurs agents cosmétiquement actifs, ce ou ces agents pouvant être présents dans les granulés et/ou hors des granulés. Au titre de tels agents cosmétiquement actifs, on peut citer un extrait de racine d'ortie pour son action purifiante et séborégulatrice ou un extrait sec d*'Alpinia galanga,* présentant une action antipelliculaire.

La composition cosmétique selon l'invention peut en outre répondre à l'une ou plusieurs des caractéristiques décrites ci-après, mises en œuvre isolément ou en chacune de leurs combinaisons techniquement opérantes.

Dans la suite de la description, on utilise le terme « granulé » pour désigner les granulés délitables contenus à au moins 50 % en volume dans la composition selon l'invention et contenant un mélange d'au moins un tensioactif anionique, au moins un agent épaississant tel que défini ci-avant et au moins un agent absorbant tel que défini ci-avant, étant entendu que la composition selon l'invention peut contenir également d'autres types de granulés de composition autre, qui ne seront pas particulièrement décrits dans la présente description.

Selon des modes particuliers de réalisation, la composition comprend au moins 60% en volume, par rapport au volume total de la composition, des granulés.

Selon des modes particuliers de réalisation, la composition comprend au moins 70% en volume, par rapport au volume total de la composition, des granulés.

Selon des modes particuliers de réalisation, la composition comprend au moins 75% en volume, par rapport au volume total de la composition, des granulés.

Selon des modes particuliers de réalisation, la composition comprend au moins 80% en volume, par rapport au volume total de la composition, des granulés.

Dans des modes de réalisation particuliers de l'invention, les granulés présentent une taille, mesurée par tamisage, pouvant être manuel ou non, comprise entre 315 et 2000 µm, et de façon encore plus avantageuse comprise entre 315 et 1250 µm.

Dans d'autres modes de réalisation particuliers de l'invention, la taille des granulés selon l'invention est comprise entre 300 et 2000 µm.

La taille des granulés peut autrement être déterminée via un calibreur mécanique, ainsi que par granulométrie laser.

Lorsque la taille est déterminée par tamisage, la taille est prise dans la dimension la plus petite du granulé.

De préférence, les granulés selon l'invention se présentent comme de petits objets fractionnés formés de particules solides agrégées entre elles, de formes et de tailles variables. Ils peuvent être de forme régulière ou irrégulière. Ils peuvent être en particulier de forme sphérique, carrée, rectangulaire, ou allongée tels des bâtonnets.

Les granulés selon l'invention sont tels qu'ils se délitent lentement. Cette caractéristique permet aux granulés selon l'invention de développer leur activité exfoliante dans les premières minutes après leur application sur la peau ou les fibres kératiniques, avant leur désagrégation totale.

Dans des modes de mise en œuvre particuliers de l'invention, la composition contient une quantité de tensioactif(s) anionique(s) comprise entre 1 et 50 % en poids, de préférence comprise entre 5 et 40 % en poids, par rapport au poids total de la composition. Le ou les tensioactifs anioniques peuvent être présents uniquement à l'intérieur des granulés, ou être également présents dans la composition hors des granulés, par exemple sous forme de poudre pulvérulente.

Les granulés selon l'invention comprennent au moins un, le cas échéant plusieurs, tensioactif(s) anionique(s), qui apporte(nt) avantageusement le caractère nettoyant, ainsi que moussant, à la composition. Ce ou ces tensioactifs anioniques sont de préférence avantageusement dispersibles dans l'eau. Ce ou ces tensioactifs anioniques peuvent être choisis par exemple parmi les carboxylates, les sulfonates, les phosphates, les iséthionates, les sarcosinates, les glutamates, les lactilates, les taurates, les sels d'acide gras, les sels de galactoside uroniques, les sels d'acides éther carboxyliques et leurs mélanges.

Selon des modes de réalisation de l'invention, un tensioactif convenant particulièrement à l'invention peut être choisi parmi les dérivés d'acide iséthionique et leurs sels.

Ce ou ces tensioactifs anioniques peuvent être par exemple choisis parmi les acides acyl iséthioniques, leurs sels (iséthionates) et leurs mélanges, de préférence parmi les sels d'acide acyl iséthionique dont la chaine hydrocarbonée R₁ du groupement acyl R₁C=O, est linéaire ou ramifiée, saturée ou insaturée, et comprend de 8 à 30 atomes de carbone, de préférence de 10 à 20 atomes de carbone, de façon préférée de 12 à 18 atomes de carbone. Le groupe acyl peut être notamment choisi parmi les groupes lauroyl, myristoyl, palmitoyl, stearoyl, olivoyl, cocoyl, oleoyl et leurs mélanges.

Par « leurs sels » on entend que l'atome d'hydrogène de la fonction acide de l'acide iséthionique est remplacé par un cation M⁺, par exemple choisi parmi les ions des métaux alcalins tels que Na, Li, K, de préférence Na ou K, parmi les ions des métaux alcalino-terreux tels que Mg, les groupes ammonium et leurs mélanges.

A titre d'exemples de dérivés d'acide iséthionique utilisables dans le cadre de l'invention, on peut citer par exemple le composé de nom INCI sodium lauroyl methyl isethionate comme les références Iselux^{®} LQ-CLR, Iselux^{®} LQ-CLR-SB, ou le mélange sodium lauroyl methyl isethionate / sodium methyl isethionate tel que l'Iselux^{®} proposé par la société Innospec. On peut également citer le composé portant le nom INCI sodium cocoyl isethionate comme les références Hostapon^{®} SCI commercialisé par la société Clariant ou Pureact I-85EC commercialisé par la société Innospec Active Chemicals. On peut également citer le cocoyl-iséthionate de sodium commercialisé sous la dénomination Jordapon^{®} CI par la société BASF. Un tensioactif anionique préféré dans le cadre de l'invention est le cocoyl-iséthionate de sodium tel que commercialisé par la société Akzo Nobel sous la dénomination Elfan^{®} AT84G.

Selon des modes préférés de réalisation de l'invention, les granulés selon l'invention, comprennent un seul tensioactif anionique, de préférence dérivé de l'acide iséthionique. De préférence, la composition cosmétique selon l'invention est dépourvue de tensioactif anionique sulfaté. Par l'expression « un tensioactif anionique sulfaté », on entend un tensioactif anionique comprenant au moins une fonction sulfate (-OSO₃H ou -OSO₃⁻), et pouvant éventuellement comprendre en outre une ou plusieurs autres fonctions dérivées d'acides, telles que les fonctions acides carboxyliques ou carboxylates (-COOH) ou - COO⁻), les fonctions sulfonates (-SO₃H ou -SO₃⁻) et/ou les fonctions phosphates.

La composition cosmétique selon l'invention peut contenir, en plus du tensioactif anionique ou à la place du tensioactif anionique, dans et/ou hors des granulés, un ou plusieurs tensioactifs non ioniques. Ce ou ces tensioactifs non ioniques peuvent par exemple être choisis parmi les alkyl polyglucosides.

Dans des modes de réalisation particuliers de l'invention, la quantité totale de tensioactifs contenus dans la composition, en particulier dans les granulés délitables, est comprise entre 1 et 50 % en poids par rapport au poids total de la composition. Une telle caractéristique, associée à la présence dans les granulés d'au moins un agent épaississant choisi parmi les gommes, la quantité totale de gommes dans la composition étant comprise entre 1 et 4 % en poids par rapport au poids total de la composition, permet avantageusement de former, au contact de l'eau, une mousse d'abondance optimale concernant les performances lavantes de la composition et la facilité de rinçage.

Les granulés selon l'invention comprennent au moins un agent absorbant, qui permet notamment d'éliminer l'excès de sébum sur la peau et/ou les fibres kératiniques. Cet agent absorbant peut être d'origine minérale ou organique.

Selon des modes de réalisation de l'invention, l'agent absorbant est dispersible dans l'eau.

L' agent absorbant est choisi parmi les amidons.

Les amidons utilisables dans la présente invention sont, par exemple, l'amidon de maïs, l'amidon de pomme de terre, l'amidon de pois, l'amidon de riz, l'amidon d'avoine, l'amidon d'orge, l'amidon de tapioca, l'amidon de blé, l'amidon de cassaya, et le sorgo.

Selon des modes de réalisation particuliers de l'invention, la composition cosmétique contient au moins un agent absorbant sous forme de poudre blanche, dont la taille des particules élémentaires varie de 15 à 100 µm, cet agent absorbant étant présent dans et/ou hors des granulés.

Selon des modes de réalisation de l'invention, les granulés contiennent au moins, dans et/ou hors des granulés, trois agents absorbants choisis parmi les amidons et les argiles.

Selon des modes de réalisation de l'invention, la composition selon l'invention contient au moins, dans et/ou hors des granulés, deux agents absorbants choisis parmi les amidons et les argiles.

Selon d'autres modes de réalisation de l'invention, la composition selon l'invention ne contient qu'un seul agent absorbant.

Le ou les agents absorbants peuvent être présents dans la composition selon l'invention en une quantité totale allant de 1 à 80% en poids, de préférence de 2 à 60 % en poids et préférentiellement de 20 à 60% en poids, par rapport au poids total de la composition.

Les granulés selon l'invention comprennent un ou plusieurs agents épaississants choisis parmi les gommes d'origine végétale, les gommes d'origine microbienne et/ou les gommes d'origine synthétique. Les gommes d'origine végétale sont extraites des arbres, d'arbustes feuillus et des algues. Les gommes d'origine microbiennes sont obtenues par synthèse de ces macromolécules par des souches sélectionnées de micro-organismes en conditions contrôlées dans les fermenteurs. Quant aux gommes synthétiques, elles sont obtenues par modification ou dérivation des gommes naturelles et par synthèse chimique. Les gommes d'origine végétale peuvent être choisies parmi la gomme arabique, la gomme ghatti, la gomme karaya, les pectines, la gomme guar, la gomme caroube, l'agar, les alginates, les carraghénanes,

Les gommes d'origine microbiennes peuvent être choisies parmi la gomme de scléroglucane, la gomme de gellane, la gomme de pullulane, la gomme de Curdlar, la gomme de xanthane, la gomme de grifolane, la gomme de lentinane, la gomme de Schizophyllane, la gomme de spirulinane et la gomme de krestine.

Les gommes d'origine synthétique peuvent être choisies parmi les dérivés de cellulose.

Selon des modes de réalisation de l'invention, la composition selon l'invention contient deux agents épaississants qui sont de préférence choisis parmi les gommes d'origine végétale et/ou d'origine microbienne.

De façon préférée, des gommes particulièrement intéressantes dans la présente invention sont la gomme arabique et/ou la gomme xanthane.

Le ou les agents épaississants sont présents dans la composition selon l'invention en une quantité totale allant de 1 à 4 % en poids, de préférence de 2 à 4 % en poids, et préférentiellement de 2 à 3 % en poids, par rapport au poids total de la composition. Au sein de la composition, ils sont de préférence uniquement contenus dans les granulés délitables.

Selon des modes de réalisation particuliers de l'invention, la composition selon l'invention comprend en outre un agent exfoliant, qui y est de préférence présent en dehors des granulés, et qui va permettre le gommage de la peau (dont le cuir chevelu), par son action mécanique. L'agent exfoliant peut être choisi parmi les agents d'origines minérale, végétale, ou organique. Ainsi, on peut utiliser par exemple les billes ou la poudre de polyéthylène, les particules de nylon, la poudre de polychlorure de vinyle, la pierre ponce (nom INCI : pumice), les coques de noix, la sciure de bois, la farine de bois, la farine de liège, les billes de verre, l'alumine (oxyde d'aluminium) (nom INCI : Alumina), les cristaux de sucre, des billes qui fondent lors de l'application sur la peau, telles que par exemple, les sphères à base de mannitol et cellulose, les capsules à base d'agar et les sphères à base d'esters de jojoba, et leurs mélanges. Ce ou ces agents exfoliants ne sont notamment préférentiellement pas solubles dans l'eau.

Selon des modes de réalisation de l'invention, la composition cosmétique selon l'invention est dénuée de sulfate, afin d'améliorer sa tolérance.

Selon des modes de réalisation de l'invention, la composition cosmétique selon l'invention est dénuée de silice, afin d'améliorer sa tolérance.

Selon des modes de réalisation de l'invention, la composition cosmétique selon l'invention est dénuée de talc, afin d'améliorer sa tolérance.

Selon des modes de réalisation de l'invention, la composition cosmétique selon l'invention est dénuée de sulfate, de silice et de talc.

La composition cosmétique selon l'invention peut en outre contenir un ou plusieurs additifs, pouvant être présents dans et/ou hors des granulés, et par exemple choisis parmi les polymères anioniques, cationiques, non ioniques, amphotères, conditionneurs ou fixants, les parfums, les colorants, les filtres protecteurs, les acides, les bases, les nacres, les paillettes, etc.

Ces additifs peuvent être présents dans la composition selon l'invention en une quantité allant de 0 à 20% en poids par rapport au poids total de la composition.

L'homme du métier veillera à choisir ces éventuels additifs et leurs quantités de manière à ce qu'ils ne nuisent pas aux propriétés de la composition de la présente invention.

Dans des modes de réalisation particuliers de l'invention, les granulés contiennent une quantité de polyol(s), notamment de propylène glycol, inférieure à 2 % en poids par rapport au poids total de la composition, de préférence inférieure à 1 % en poids et préférentiellement inférieure à 0,5 % en poids, par rapport au poids total de la composition.

Selon des modes de réalisation de l'invention, la composition selon l'invention se présente sous une forme adaptée pour une application topique unique. La composition cosmétique selon l'invention peut notamment être conditionnée sous forme d'unidose, par exemple dans un sachet.

Selon des modes de réalisation de l'invention, la composition cosmétique selon l'invention est conditionnée dans un sachet de 2 à 6 g, de façon préférée, de 2 à 4g, et de façon encore préférée dans un sachet de 3g.

La composition cosmétique selon l'invention peut constituer un produit de nettoyage de la peau, du corps et/ou du visage, du cuir chevelu et/ou des fibres kératiniques, préférentiellement des cheveux. Elle peut par exemple être un shampoing, un gel douche, un masque pour les cheveux ou la peau, etc.

Elle peut également être un produit de gommage, c'est-à-dire un produit qui sert à éliminer les cellules mortes de l'épiderme, la peau (dont le cuir chevelu) étant ainsi lissée, et le renouvellement cellulaire étant donc favorisé. Son action exfoliante est d'autant plus importante lorsque, conformément à des modes de réalisation particuliers de l'invention, elle contient un agent exfoliant, hors des granulés.

Selon un deuxième aspect, l'invention concerne un procédé de lavage et/ou de traitement cosmétique, non thérapeutique, des fibres kératiniques, notamment humaines, en particulier des poils et des cheveux. Ce procédé comporte une étape d'application, sur les fibres kératiniques humides, d'une composition cosmétique selon l'invention, telle que définie ci-avant, suivie optionnellement d'un massage pour développer la mousse, par contact de l'eau avec le tensioactif anionique contenu dans les granulés de la composition, puis d'un temps de pose optionnel pouvant de préférence aller jusqu'à 5 minutes, puis enfin d'un rinçage à l'eau desdites matières kératiniques.

Selon des modes de mise en œuvre particuliers de l'invention, de la mousse est développée préalablement à l'étape d'application de la composition sur les fibres kératiniques, en versant la composition selon l'invention dans la main puis en ajoutant de l'eau, la composition moussante ainsi obtenue étant ensuite appliquée sur le cuir chevelu et/ou les cheveux pour le lavage, puis une étape de rinçage après un temps de pose optionnel.

Selon un autre aspect, l'invention concerne un procédé de lavage et/ou de traitement cosmétique, non thérapeutique, de la peau, notamment humaine, ce procédé comportant une étape d'application, sur la peau humide, d'une composition cosmétique selon l'invention, telle que définie ci-avant, suivie optionnellement d'un massage pour développer la mousse, par contact de l'eau avec le tensioactif anionique contenu dans les granulés de la composition, puis d'un temps de pose optionnel pouvant de préférence aller jusqu'à 5 minutes, puis enfin d'un rinçage de la peau à l'eau.

Là encore, de la mousse peut être développée préalablement à l'étape d'application de la composition sur la peau, en versant la composition selon l'invention dans la main puis en ajoutant de l'eau, la composition moussante ainsi obtenue étant ensuite appliquée sur la peau pour le lavage, puis une étape de rinçage après un temps de pose optionnel.

Un autre aspect de l'invention concerne l'utilisation cosmétique, non thérapeutique, pour le gommage de la peau du visage et/ou du corps d'un individu, en particulier humain, d'une composition cosmétique selon l'invention, telle que définie ci-avant.

Selon un autre aspect, la présente invention concerne un granulé délitable destiné à être mis en œuvre dans une composition cosmétique, convenant à une application topique sur la peau et/ou les fibres kératiniques, notamment les cheveux, d'un individu, notamment un individu humain, ce granulé présentant une taille, mesurée par tamisage, comprise entre 150 et 2000 µm et contenant un mélange de :
- au moins un tensioactif anionique,
- au moins un agent épaississant choisi parmi les gommes d'origine végétale, microbienne et/ou synthétique,
- et au moins un agent absorbant choisi parmi les amidons
la quantité totale du ou des agents épaississants choisis parmi les gommes d'origine végétale, microbienne et/ou synthétique, dans le granulé, étant comprise entre 1 et 4 % en poids, de préférence entre 2 et 4 % en poids, par rapport au poids total du granulé.

Ce granulé peut répondre à l'une ou plusieurs des caractéristiques se rapportant aux granulés énoncées ci-avant dans le cadre de la description de la composition cosmétique selon l'invention.

En particulier, il peut présenter une taille, mesurée par tamisage, comprise entre 315 et 1250 µm.

Il peut en outre contenir 1 à 50 % en poids, de préférence de 5 à 50 % en poids et préférentiellement de 5 à 40 % en poids, par rapport au poids total du granulé, de tensioactif(s) anionique(s).

Dans des modes de réalisation particuliers de l'invention, le granulé comprend une quantité totale de tensioactifs comprise entre 1 et 50 % en poids par rapport au poids total dudit granulé.

Un autre aspect est décrit présentement et concerne un procédé de préparation d'un granulé délitable selon l'invention, par granulation humide. Ce procédé comprend des étapes successives de :
- préparation d'un mélange contenant ledit au moins un tensioactif anionique, ledit au moins un agent absorbant et ledit au moins un agent épaississant, en quantité comprise entre 1 et 4 % en poids, de préférence entre 2 et 4 % en poids, par rapport au poids total du granulé,
- ajout d'un liquide de mouillage à ce mélange,
- formation d'un granulé, par exemple par cisaillement mécanique ou pulvérisation,
- et séchage de ce granulé, de préférence de sorte à obtenir une quantité de liquide de mouillage résiduelle inférieure ou égale à 0,5 % en poids par rapport au poids total du granulé.

Préférentiellement, le liquide de mouillage est de type aqueux. Il contient de préférence plus de 50 % en poids d'eau, de préférence plus de 80 % en poids d'eau.

Dans des modes de mise en œuvre particuliers de l'invention, le liquide de mouillage est formé uniquement d'eau. Dans des modes de mise en œuvre alternatifs, il comprend, outre de l'eau, un ou plusieurs ingrédients cosmétiquement acceptables hydrosolubles, tels que des actifs cosmétiques hydrosolubles, par exemple un extrait de racine d'ortie, et/ou des émollients hydrosolubles.

Préférentiellement, le liquide de mouillage contient moins de 2 % en poids, de préférence moins de 1 % en poids, et préférentiellement 0 % en poids, de polyol(s), en particulier de propylène glycol.

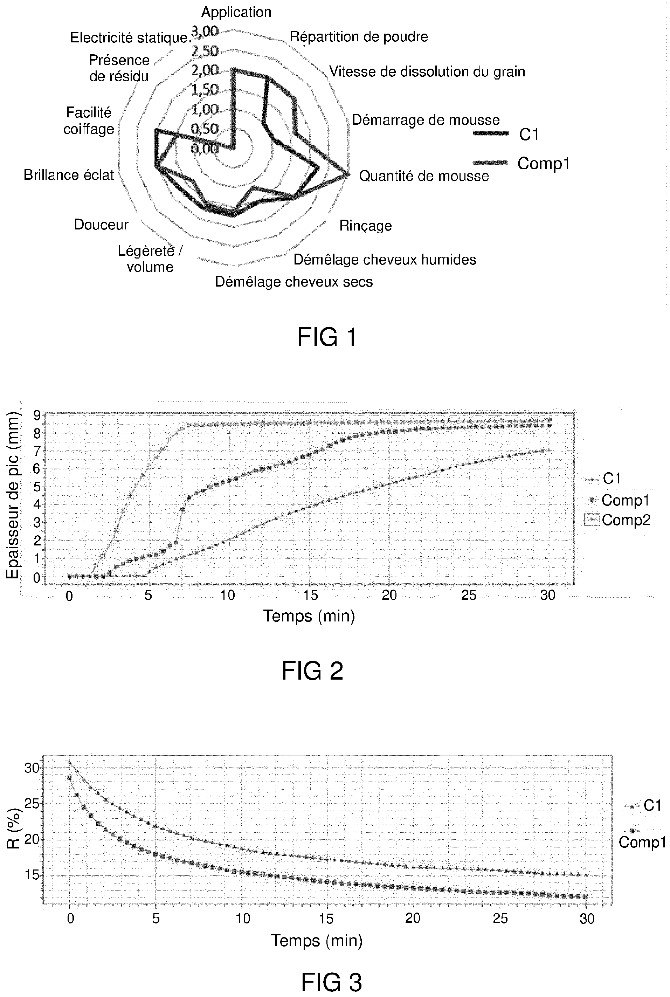

Les caractéristiques et avantages de l'invention apparaîtront plus clairement à la lumière des exemples de mise en œuvre ci-après, fournis à simple titre illustratif et nullement limitatifs de l'invention, avec l'appui des figures 1 à 3, dans lesquelles :
- la figure 1 montre les profils d'analyse sensorielle d'une composition cosmétique pour le soin des cheveux conforme à l'invention C1 et d'une composition comparative non conforme à l'invention, dépourvue de gomme, Comp1 ;
- la figure 2 montre un graphe illustrant, en fonction du temps, l'épaisseur du pic mesurée lors d'un essai d'analyse de stabilité physique Turbiscan^{®} après mélange dans l'eau et agitation pour provoquer la formation de mousse, pour une composition cosmétique pour le soin des cheveux conforme à l'invention C1 et des compositions comparatives non conformes à l'invention, dépourvues de gomme, Comp1 et Comp2 ;
- et la figure 3 montre un graphe illustrant, en fonction du temps, la valeur moyenne de rétrodiffusion mesurée lors d'un essai d'analyse de stabilité physique Turbiscan^{®} après mélange dans l'eau et agitation pour provoquer la formation de mousse, pour une composition cosmétique pour le soin des cheveux conforme à l'invention C1 et une composition comparative non conforme à l'invention, dépourvue de gomme, Comp1.

### Exemple 1 : Formulation de composition selon l'invention

Une composition cosmétique sous forme sèche conforme à l'invention répond à la composition indiquée dans le tableau 1 ci-dessous.

**Tableau 1 - Composition cosmétique selon l'invention**

| Composants | Quantité (% en poids) |
|---|---|
| Sodium cocoyl isethionate | 5 - 50 |
| Acide gras de coco | 0,5 - 5 |
| Amidon de maïs | 1 - 70 |
| Amidon de riz | 1 - 70 |
| Hectorite | 2 - 10 |
| Argile blanche (Kaolin) | 2 - 10 |
| Gomme arabique | 0,5 - 2 |
| Gomme xanthane | 0,5 - 2 |
| Extrait sec de Bambou | 0,5 - 2 |
| Laurate de glycéryle | 0,6 - 1 |
| Parfum | 0,5 - 1 |
| Propylène glycol | 0 - 1 |
| Extrait de racine de Grande Ortie | 0 - 0,5 |
| Eau | 0 - 0,5 |

### Exemple 2 : Procédé d'obtention de granulés selon l'invention

Les granulés selon l'invention sont obtenus par une étape de granulation, plus particulièrement de granulation humide.

La granulation a pour but de transformer des particules de poudres cristallisées ou amorphes en agrégats solides plus ou moins résistants et plus ou moins poreux appelés granulés ou grains après calibrage.

Les avantages des granulés selon l'invention, obtenus par granulation, sont multiples :
- une meilleure conservation de l'homogénéité du mélange de constituants. La granulation va en effet provoquer l'apparition de liaisons entre les particules des différents constituants, ces liaisons pouvant contribuer à l'obtention et au maintien de l'homogénéité du mélange ;
- un bon écoulement de la composition les contenant. Cette amélioration des propriétés d'écoulement du mélange pourrait notamment être due à l'augmentation de la densité et de la taille des particules par la formation d'un granulé, et à la modification de la forme et de l'état de surface de ces particules ;
- une réduction du dégagement de poussières comparativement au même mélange à l'état pulvérulent. La forme en granulés participe ainsi notamment à la diminution des problèmes de contaminations croisées et facilite les nettoyages lors de la fabrication et du conditionnement de la composition ;
- la maitrise de la vitesse de dissolution/dispersion dans l'eau des constituants des granulés. En effet, l'emploi de liants, tels que l'agent absorbant contenu dans les granulés selon l'invention, qui sont le plus souvent des substances de nature hydrophile, lors de la granulation, peut entrainer une amélioration du mouillage des surfaces et par là-même améliorer notamment la vitesse de dissolution dans l'eau des constituants hydrophobes.

La granulation humide consiste en l'agglomération de poudres à l'aide d'un liquide de mouillage. On distingue les étapes suivantes :
- mélange des matières premières : cette étape a pour but de répartir les constituants de façon homogène ;
- fabrication d'une solution de liant et transfert dans le granulateur ;
- mouillage des poudres et mélange ; cette étape a pour but de créer des liaisons entre les particules, celles-ci devant être suffisamment fortes pour que l'ensemble supporte l'opération de granulation proprement dite ;
- granulation, cette étape ayant pour but de mélanger poudre et liquide pour atteindre la structure granuleuse, par cisaillement mécanique ou par pulvérisation ;
- répartition sur plateau des granulés humides ; cette étape n'est présente que lorsqu'un séchage en étuve ultérieur est prévu ;
- séchage, cette étape permettant de diminuer le taux d'humidité à une valeur adaptée pour éviter la dégradation des constituants et permettre la mise en forme des granulés ;
- calibrage, permettant d'obtenir une bonne répartition granulométrique par passage au travers d'une grille calibrée.

Afin de s'assurer de la conformité des granulés obtenus, on peut avoir recours à plusieurs méthodes de contrôle concernant plusieurs paramètres : la granulométrie, la densité apparente, l'écoulement, la porosité, la dureté, l'humidité résiduelle, la désagrégation, la dissolution.

Dans cet exemple, le mode opératoire précis suivant a été utilisé, pour une taille d'un lot de 210 kg, dans un mélangeur de type Lödige de 600 litres :
- mélanger les poudres qui constituent les granulés pendant 3 minutes (dans cet exemple, tous les ingrédients indiqués dans le tableau 1 ci-dessus, à l'exception de l'hectorite, le kaolin et le parfum) ;
- mouiller les poudres en pulvérisant 3 fois 10 litres d'eau en 3 fois 3 minutes tout en mélangeant ;
- sécher les granulés humides en étuve à plateaux à 75°C jusqu'à obtenir le pourcentage d'humidité relative visé, de préférence inférieur à 4% tel que mesuré par un dessiccateur halogène pour 2 g pendant 10 minutes à 105°C ;
- calibrer les grains secs sur une grille de 1,25 mm par exemple ;

Aux granulés ainsi obtenus sont ajoutés les autres ingrédients qui constituent la composition cosmétique (dans cet exemple, poudre d'hectorite, poudre de kaolin et parfum, ce dernier étant absorbé sur les poudres d'hectorite et de kaolin), et réaliser le mélange final, les granulés constituant plus de 50 % en volume de la composition.

### Exemple 3 : Caractérisation de la composition cosmétique et des granulés selon l'invention

L'objectif de cette étude est de caractériser différents échantillons de la composition cosmétique selon l'invention et des granulés qui entrent dans sa constitution.

Les techniques mises en œuvre sont :
- le tamisage, qui permet de déterminer la distribution granulométrique de l'échantillon ;
- le Densi-Tap^{™}, qui permet de déterminer la densité apparente et la densité tassée des poudres et des granulés (répondant à la norme ASTM D4164).
- le débitmètre à entonnoir de Hall/Carney (cône de Carney), qui permet de caractériser pour chaque échantillon l'aptitude à l'écoulement par détermination du temps d'écoulement.

Les échantillons étudiés sont les suivants :
- Composition comprenant les composants indiqués dans le tableau 1 ci-dessus sous forme de poudres distinctes (exemple comparatif de composition non conforme à l'invention) ;
- Granulés formés comme décrit dans l'exemple 2 ;
- Composition finale, conforme à l'invention, contenant ces granulés et les ingrédients supplémentaires, comme décrit dans l'exemple 2.

### Techniques analytiques

### La distribution granulométrique par tamisage

La distribution granulométrique par tamisage a été réalisée. Les tamis ont été adaptés en fonction des échantillons. Pour l'échantillon poudre, cinq tamis de taille 25µm, 53µm, 75µm, 150µm et 315µm ont été sélectionnés. Pour les échantillons granulés, cinq tamis de taille 75µm, 150µm, 315µm, 600µm et 1mm ont été sélectionnés. Le tamisage a été effectué jusqu'à obtenir une masse constante par gamme de taille.

### La densité apparente et densité tassée

Technique de caractérisation : volumétrie de tassage
Appareillage : Stampfvolumeter STAV 2003 JEL
Principe : La méthode consiste à provoquer le tassement d'une poudre placée dans une éprouvette normalisée, en lui faisant subir une suite de chocs standardisés. La masse volumique tassée est déterminée à la suite de 200 chocs. La masse volumique est calculée selon la formule : ρ = m/V, où m est la masse en poudre en g et V le volume de poudre en cm³.

### La coulabilité

L'évaluation de la coulabilité selon la méthode du cône de Carney consiste à déterminer le temps d'écoulement des poudres métalliques à travers un entonnoir calibré d'une ouverture de 5mm. Les mesures sont réalisées sur des prises d'essai de 25ml. Le temps d'écoulement de la totalité de la prise d'essai est mesuré. Le temps d'écoulement est calculé sur une moyenne de trois essais par échantillon.

### Résultats

Les résultats obtenus pour la distribution granulométrique par tamisage sont présentés dans les tableaux 2 (pour le mélange de poudres) et 3 (pour les granulés et la composition selon l'invention).

**Tableau 2 - distribution granulométrique du mélange de poudres L'échantillon sous forme de poudre présente une distribution granulométrique centrée sur 53-75 µm.**

| Taille | Distribution granulométrique (%) de la forme poudre |
|---|---|
| < 25µm | 6,1 |
| 25-53 µm | 25,4 |
| 53-75 µm | 42,2 |
| 75-150 µm | 22,8 |
| 150-315 µm | 0,8 |
| > 315 µm | 2,7 |

**Tableau 3 - distribution granulométrique des granulés et de la composition complète selon l'invention**

| | Distribution granulométrique (%) | |
|---|---|---|
| Taille | Granulés | Composition complète |
| < 75 µm | 5,8 | 14,8 |
| 75-150 µm | 5,5 | 6,4 |
| 150-315 µm | 17,2 | 13,9 |
| 315-600 µm | 32,3 | 28,9 |
| 600-1000 µm | 30,7 | 28,8 |
| >1 mm | 8,5 | 7,2 |

Les échantillons granulés présentent une distribution granulométrique dispersée et centrée sur 315 µm à 1 mm. Il est à noter que l'échantillon « composition complète » est constitué de particules fines de taille inférieure à 75 µm en plus grande quantité que l'échantillon « granulés ». Environ 80 % de la formule complète se présente sous forme de granulés ayant une taille supérieure à 150 µm.

Les résultats de la densité apparente et de la densité tassée sont présentés dans le tableau 4 ci-après, ces résultats étant issus de la moyenne de trois mesures.

**Tableau 4 - Caractéristiques du mélange de poudres et des granulés selon l'invention**

| Echantillons | Masse volumique apparente (g/cm³) | Masse volumique tassée (g/cm³) |
|---|---|---|
| Forme poudres | 0,41 ± 0,01 | 0,56 ± 0,01 |
| Granulés | 0,66 ± 0,01 | 0,76 ± 0,01 |

Les échantillons sous forme de granulés présentent une densité apparente beaucoup plus élevée que l'échantillon non granulé, mélange de poudres. Les échantillons granulés sont également moins sensibles au tassement, signe d'une cohésion plus faible et d'un écoulement potentiellement meilleur.

Les résultats de coulabilité sont présentés dans le tableau 5.

**Tableau 5 - Résultats de coulabilité**

| | Coulabilité (s) | | |
|---|---|---|---|
| Essais | Forme poudre | Granulés | Composition complète |
| N° 1 | Pas d'écoulement | 14,76 | 14,92 |
| N° 2 | | 14,28 | 14,16 |
| N° 3 | | 14,32 | 14,15 |
| Moyenne | | 14,45 | 14,41 |

L'échantillon sous forme de simple mélange de poudres ne s'est pas écoulé à travers le système utilisé. En revanche les échantillons sous forme de granulés ont des temps d'écoulement similaires de l'ordre d'une quinzaine de secondes.

En conclusion, les analyses mises en œuvre dans cette étude ont permis de montrer premièrement que la granulation humide a été efficace, en effet le passage d'une forme poudre à une forme en granulés permet d'augmenter fortement la coulabilité. Deuxièmement, environ 80% de la composition complète se présente bien sous forme de granulés ayant une taille supérieure à 150 µm.

### Exemple 4 : Test consommateur d'une composition cosmétique selon l'invention

Le but de ce test est de caractériser les perceptions et les ressentis lors de l'utilisation de la composition selon l'invention telle que préparée à l'exemple 2 et d'en déterminer les forces et faiblesses dans des conditions réelles d'utilisation.

Les consommateurs sont interrogés en France et évaluent le produit chez eux.

96 consommateurs ont été recrutés pour ces tests, ils sont âgés de 19 à 63 ans (moyenne 39 ans), 77% d'entre eux sont des femmes. Le produit est évalué anonymement, sur une période d'utilisation test de 2 mois. Cinq dates d'évaluation sont requises : J0, J1, J30, J60 et J75.

Le protocole d'utilisation est le suivant :
- Verser la composition sous forme de granulés dans la paume de la main préalablement humidifiée ;
- Appliquer sur les cheveux mouillés ;
- Masser afin de former une mousse ;
- Laisser sur les cheveux pendant 1 à 2 minutes ;
- Rincer.

Pour de meilleurs résultats, il convient d'utiliser cette composition une fois par semaine pendant 2 mois.

Quantification des résultats : Evaluation sur une échelle de 0 à 10, avec 0 : je n'aime pas du tout et 10 : j'aime beaucoup ou 0 : totalement en désaccord et 10 : totalement d'accord.

### Résultats

En appréciation globale de la composition cosmétique selon l'invention, le score immédiat d'appréciation sur 10 est de 6,97, il est de 6,94 à J30 et atteint 7,00 à J60. Pour le critère de transformation de la poudre en mousse, le score à J30 par exemple atteint 7,51 sur 10 avec 81% des réponses dans les 5 notes les plus élevées (6 à 10/10). Sur la quantité de mousse, 60% des consommateurs trouvent la quantité suffisante.

De même sur la quantité de poudre par utilisation, 66% des consommateurs la trouvent suffisante.

Pour l'efficacité de nettoyage de la composition selon l'invention, le score de propreté sur 10 atteint 7,70 pour un effet immédiat, 7,62 à J30, 7,69 à J60 et 7,44 à J75. Environ 50% des consommateurs donnent la note d'au moins 8/10.

En revanche, sur le fait de ressentir ses cheveux gras, avant l'utilisation de la composition selon l'invention le score sur 10 atteint 5,67 et tombe à 1,73 immédiatement après l'utilisation de la composition selon l'invention, montrant l'efficacité de ladite composition.

Des résultats similaires sont retrouvés en se focalisant sur le cuir chevelu.

Enfin, sur la question de la comparaison de la composition selon l'invention versus le produit habituel, 38% des consommateurs la trouvent beaucoup mieux, 23% un peu mieux, et seulement 1% des consommateurs la trouvent vraiment moins bien.

Ce test consommateur a permis d'évaluer qualitativement la composition cosmétique sous forme sèche selon l'invention. Il apparait qu'elle se mélange bien à l'eau et que les granulés se désagrègent aisément. Son application sur la peau et les cheveux est facile. Elle présente de bonnes propriétés moussantes, le démarrage de mousse est satisfaisant, et l'on obtient une mousse adéquate et suffisamment abondante, et la qualité de la mousse est bonne, notamment la mousse est onctueuse et abondante.

### Exemple 5 : Comparatifs avec et sans agent épaississant

Dans cet exemple, une composition cosmétique C1 conforme à l'invention est comparée à une composition cosmétique comparative Comp1 non conforme à l'invention, de constitution identique, mais dénuée de gomme.

Ces compositions sont obtenues comme décrit dans l'exemple 2, avec les constituants dans les proportions indiquées dans le tableau 6 ci-dessous.

**Tableau 6**

| Ingrédient | C1 - quantité (% p/p) | Comp1 - quantité (% p/p) |
|---|---|---|
| Sodium cocoyl isethionate | 45 | 45 |
| Amidon de maïs | 35 | 37 |
| Amidon de riz | 16 | 16 |
| Gomme arabique | 1 | - |
| Gomme xanthane | 1 | - |
| Extrait sec de Bambou | 1 | 1 |
| Conditionneur capillaire | 1 | 1 |

Une analyse sensorielle, selon les critères suivants, a été réalisée pour chacune de ces compositions, en appliquant le protocole décrit dans l'exemple 4 ci-dessus, sur cheveux mouillés : application, répartition de la poudre, vitesse de dissolution des grains, démarrage de mousse, quantité de mousse, rinçage, démêlage des cheveux humides, démêlage des cheveux secs, légèreté / volume, douceur, brillance / éclat, facilité de coiffage, présence de résidus après rinçage, électricité statique. Les résultats obtenus sont montrés sur la figure 1.

On observe que, contrairement à la composition Comp1, pour la composition selon l'invention C1 :
- le démarrage de mousse est moins rapide - la transformation s'effectue par étapes : la poudre se transforme d'abord en lait onctueux de massage avec encore présence de granulés non dissous, puis on observe une transformation totale en mousse - l'action exfoliante est accentuée en début d'utilisation, par rapport à la composition comparative Comp1 ;
- la quantité de mousse est moins importante - cette quantité est néanmoins jugée suffisante par une grande majorité des utilisateurs - si bien qu'une quantité moindre d'eau est nécessaire pour le rinçage ;
- la sensation de douceur est plus importante.

Ces avantages ne sont pas obtenus au détriment des effets bénéfiques attendus pour les cheveux, notamment concernant la facilité de coiffage, qui est même au contraire améliorée, notamment sur l'aspect démêlage des cheveux humides. Les cheveux paraissent en outre plus gainés, sans effet alourdissant.

La composition selon l'invention C1 et la composition comparative non conforme à l'invention Comp1 sont en outre soumises à un test de stabilité physique. Une autre composition comparative non conforme à l'invention, nommée Comp2, également dépourvue de gomme en tant qu'agent épaississant, est également soumise à ce test. Cette composition Comp2 répond à la composition de l'exemple 3 du document FR 3 068 243.

A cet effet, il est mis en œuvre le protocole suivant :
- dans une cellule pour appareil Turbiscan^{®}, peser 0,05g de composition et compléter jusqu'à 5mL d'eau distillée.
- fermer la cellule et secouer 50 fois de sorte à provoquer la formation de mousse.
- placer la cellule immédiatement dans l'appareil Turbiscan^{®} et lancer une analyse d'un scan toutes les 20 secondes pendant 30 minutes.

Le Turbiscan^{®} est un appareil qui envoie un faisceau lumineux sur un échantillon présent dans une cellule transparente en effectuant un balayage en partant du bas de l'échantillon vers le haut. Il peut ensuite détecter le flux lumineux transmis (qui passe à travers l'échantillon) et rétrodiffusé (qui ne passe pas à travers l'échantillon). En répétant cette mesure dans le temps à une fréquence adaptée, l'instrument permet de suivre la stabilité de l'échantillon.

Concernant le flux lumineux transmis, la mesure de l'épaisseur du pic en fonction du temps permet d'observer la vitesse de formation de la phase de drainage dans le bas de la cellule. L'augmentation de la transmission signifie que la lumière peut passer à travers l'échantillon, donc qu'il n'y a plus de bulles dans le bas de l'échantillon et qu'elles ont été remplacées par un liquide de drainage qui laisse passer la lumière, témoignant d'une destruction de la mousse. Plus la pente de la courbe est élevée et plus la déstabilisation de l'échantillon a été rapide.

Les résultats obtenus pour les 3 échantillons testés sont montrés sur la figure 2. On observe que la courbe correspondant à la composition comparative Comp2 présente une pente plus élevée que les autres, ce qui démontre que le liquide de drainage y est apparu plus rapidement. La phase de drainage est également apparue bien plus rapidement pour la composition comparative Comp1 que pour la composition conforme à l'invention C1. Ces résultats démontrent clairement que la mousse obtenue avec la composition conforme à l'invention C1 est bien plus stable que celles obtenues avec les compositions comparatives, ses bulles éclatant moins vite. Elle est notamment avantageusement très stable pendant les 5 premières minutes, à savoir pendant la durée idéale d'utilisation d'une composition nettoyante pour fibres kératiniques. Cette stabilité assure notamment une efficacité nettoyante optimale.

Concernant la rétrodiffusion, elle permet également de déterminer la vitesse à laquelle les bulles éclatent dans le milieu de la cellule. Une rétrodiffusion élevée signifie que les bulles ne laissent pas passer la lumière à travers l'échantillon ; au contraire une rétrodiffusion plus faible signifie qu'il n'y a plus de bulles, car la lumière peut passer à travers la cellule.

Les résultats obtenus sont montrés sur la figure 3, pour la composition conforme à l'invention C1 et la composition comparative Comp1. On observe que la pente de la courbe correspondant à cette dernière est plus élevée, témoignant d'une moins bonne stabilité de la mousse que dans le cas de la composition conforme à l'invention C1.

### Exemple 6 : Formulation de composition selon l'invention - shampoing antipelliculaire

Une composition cosmétique sous forme sèche conforme à l'invention répond à la constitution indiquée dans le tableau 7 ci-dessous.

**Tableau 7**

| Composants | Quantité (% en poids) |
|---|---|
| Sodium cocoyl isethionate | 25 - 50 |
| Amidon de maïs | 30 - 50 |
| Silicate d'aluminium | 10 - 30 |
| Argile verte | 2 - 10 |
| Gomme arabique | 0,5 - 2 |
| Gomme xanthane | 0,5 - 2 |
| Parfum | 0,5 - 1 |
| Conditionneur capillaire | 0,5 - 2 |
| Extrait sec d'*Alpinia galanga* | 0,3 - 1,0 |
| Eau | 0 - 0,5 |

Cette composition est préparée comme décrit dans l'exemple 2.

Elle présente des propriétés similaires à celles de la composition de l'exemple 2 et de la composition C1 de l'exemple 5 ci-dessus.

## Revendications

1. Composition cosmétique sous forme sèche, convenant à une application topique sur la peau et/ou les fibres kératiniques, comprenant au moins 50 % en volume de granulés délitables présentant une taille, mesurée par tamisage, comprise entre 150 et 2000 µm et contenant un mélange d'au moins un tensioactif anionique et au moins un agent absorbant choisi parmi les amidons, ladite composition cosmétique étant **caractérisée en ce que** lesdits granulés délitables comprennent en outre au moins un agent épaississant choisi parmi les gommes d'origine végétale, microbienne et/ou synthétique, et la quantité totale dudit ou desdits agents épaississants dans ladite composition est comprise entre 1 et 4 % en poids par rapport au poids total de la composition.

2. Composition cosmétique selon la revendication 1, comprenant au moins 75 % en volume desdits granulés.

3. Composition cosmétique selon la revendication 1 ou 2, dans laquelle la taille desdits granulés est comprise entre 315 et 1250 µm.

4. Composition cosmétique selon l'une quelconque des revendications 1 à 4, dans laquelle la quantité totale dudit ou desdits agents épaississants choisis parmi les gommes d'origine végétale, microbienne et/ou synthétique, présente dans lesdits granulés délitables, est comprise entre 2 et 4 % en poids par rapport au poids total de la composition.

5. Composition cosmétique selon l'une quelconque des revendications 1 à 4, contenant une quantité comprise entre 1 et 50 % en poids, par rapport au poids total de la composition, de tensioactif(s) anionique(s).

6. Composition cosmétique selon l'une quelconque des revendications 1 à 5, **caractérisée en ce que** ledit tensioactif anionique est choisi parmi les dérivés d'acide iséthionique et leurs sels.

7. Composition cosmétique selon l'une quelconque des revendications 1 à 6, **caractérisée en ce que** lesdits granulés comprennent en outre au moins une argile.

8. Composition cosmétique selon l'une quelconque des revendications 1 à 7, **caractérisée en ce qu'**elle comprend en outre, en dehors desdits granulés, au moins un agent exfoliant.

9. Composition cosmétique selon l'une quelconque des revendications 1 à 8, dans laquelle lesdits granulés contiennent une quantité de polyol inférieure à 2 % en poids, de préférence inférieure à 1 % en poids, par rapport au poids total des granulés.

10. Composition cosmétique selon l'une quelconque des revendications 1 à 9, comprenant un agent cosmétiquement actif.

11. Composition cosmétique selon l'une quelconque des revendications 1 à 10, **caractérisée en ce qu'**elle est conditionnée sous forme d'unidose.

12. Procédé de lavage et/ou de traitement cosmétique non thérapeutique des fibres kératiniques, comportant une étape d'application, sur lesdites fibres kératiniques humides, d'une composition cosmétique telle que définie dans l'une quelconque des revendications 1 à 11, suivie optionnellement d'un massage pour développer de la mousse, puis d'un temps de pose optionnel de 0 à 5 minutes, puis d'un rinçage à l'eau desdites fibres kératiniques.

13. Procédé de lavage et/ou de traitement cosmétique non thérapeutique de la peau, comportant une étape d'application, sur ladite peau humide, d'une composition cosmétique telle que définie dans l'une quelconque des revendications 1 à 11, suivie optionnellement d'un massage pour développer de la mousse, puis d'un temps de pose optionnel de 0 à 5 minutes, puis d'un rinçage de ladite peau à l'eau.

14. Utilisation cosmétique non thérapeutique, pour le gommage de la peau du visage et/ou du corps d'un individu, d'une composition cosmétique telle que définie dans l'une quelconque des revendications 1 à 11.

15. Granulé délitable convenant à une application topique sur la peau et/ou les fibres kératiniques d'un individu, présentant une taille, mesurée par tamisage, comprise entre 150 et 2000 µm et contenant un mélange d'au moins un tensioactif anionique, et au moins un agent absorbant choisi parmi les amidons, ledit granulé étant **caractérisé en ce qu'**il contient en outre au moins un agent épaississant choisi parmi les gommes d'origine végétale, microbienne et/ou synthétique, la quantité totale dudit ou desdits agents épaississants dans ledit granulé étant comprise entre 1 et 4 % en poids, par rapport au poids total dudit granulé.

16. Granulé selon la revendication 15, contenant 1 à 50 % en poids, par rapport au poids total du granulé, de tensioactif(s) anionique(s).

## Patentansprüche

1. Kosmetische Zusammensetzung in trockener Form, geeignet für die topische Anwendung auf der Haut und/oder Keratinfasern, umfassend mindestens 50 Vol.-% zerbrechliche Granulate mit einer Größe, gemessen durch Sieben, zwischen 150 und 2000 µm und enthaltend eine Mischung aus mindestens einem anionischen Tensid und mindestens einem absorbierenden Mittel, das aus Stärken ausgewählt ist, wobei die kosmetische Zusammensetzung **dadurch gekennzeichnet ist, dass** die zerbrechlichen Granulate ferner mindestens ein Verdickungsmittel umfassen, das aus den Gummis pflanzlichen, mikrobiellen und/oder synthetischen Ursprungs ausgewählt ist, und die Gesamtmenge des oder der Verdickungsmittel in der Zusammensetzung zwischen 1 und 4 Gew.-%, bezogen auf das Gesamtgewicht der Zusammensetzung, beträgt.

2. Kosmetische Zusammensetzung nach Anspruch 1, umfassend mindestens 75 Vol.-% der Granulate.

3. Kosmetische Zusammensetzung nach Anspruch 1 oder 2, wobei die Größe der Granulate zwischen 315 und 1250 µm liegt.

4. Kosmetische Zusammensetzung nach einem der Ansprüche 1 bis 4, wobei die Gesamtmenge des/der Verdickungsmittel(s), ausgewählt aus den Gummis pflanzlichen, mikrobiellen und/oder synthetischen Ursprungs, die in den zerbrechlichen Granulaten vorhanden ist, zwischen 2 und 4 Gew.-%, bezogen auf das Gesamtgewicht der Zusammensetzung, beträgt.

5. Kosmetische Zusammensetzung nach einem der Ansprüche 1 bis 4, enthaltend eine Menge zwischen 1 und 50 Gew.-%, bezogen auf das Gesamtgewicht der Zusammensetzung, anionischer Tenside.

6. Kosmetische Zusammensetzung nach einem der Ansprüche 1 bis 5, **dadurch gekennzeichnet, dass** das anionische Tensid aus Isethionsäurederivaten und deren Salzen ausgewählt ist.

7. Kosmetische Zusammensetzung nach einem der Ansprüche 1 bis 6, **dadurch gekennzeichnet, dass** die Granulate ferner mindestens einen Ton umfassen.

8. Kosmetische Zusammensetzung nach einem der Ansprüche 1 bis 7, **dadurch gekennzeichnet, dass** sie ferner außer dem Granulat mindestens einen Peelingwirkstoff umfasst.

9. Kosmetische Zusammensetzung nach einem der Ansprüche 1 bis 8, wobei die Granulate eine Polyolmenge von weniger als 2 Gew.-%, vorzugsweise weniger als 1 Gew.-%, bezogen auf das Gesamtgewicht der Granulate, enthalten.

10. Kosmetische Zusammensetzung nach einem der Ansprüche 1 bis 9, umfassend einen kosmetisch aktiven Wirkstoff.

11. Kosmetische Zusammensetzung nach einem der Ansprüche 1 bis 10, **dadurch gekennzeichnet, dass** sie in Form einer Eindosis verpackt ist.

12. Verfahren zum Waschen und/oder zur nichttherapeutischen kosmetischen Behandlung von Keratinfasern, umfassend einen Schritt des Auftragens einer kosmetischen Zusammensetzung gemäß einem der Ansprüche 1 bis 11 auf die feuchten Keratinfasern, optional gefolgt von einer Massage zur Entwicklung von Schaum, anschließend einer optionalen Einwirkzeit von 0 bis 5 Minuten und anschließendem Spülen der Keratinfasern mit Wasser.

13. Verfahren zum Waschen und/oder zur nichttherapeutischen kosmetischen Behandlung der Haut, umfassend einen Schritt des Auftragens einer kosmetischen Zusammensetzung gemäß einem der Ansprüche 1 bis 11 auf die feuchte Haut, optional gefolgt von einer Massage zur Entwicklung von Schaum, anschließend einer optionalen Einwirkzeit von 0 bis 5 Minuten und anschließendem Spülen der Haut mit Wasser.

14. Nichttherapeutische kosmetische Verwendung, zum Peeling der Haut des Gesichts und/oder des Körpers einer Person, einer kosmetischen Zusammensetzung gemäß einem der Ansprüche 1 bis 11.

15. Zerbrechliches Granulat, das für die topische Anwendung auf der Haut und/oder den Keratinfasern einer Person geeignet ist, mit einer Größe, gemessen durch Sieben, zwischen 150 und 2000 µm und mit einer Mischung aus mindestens einem anionischen Tensid und mindestens einem absorbierenden Mittel, das aus Stärken ausgewählt ist, wobei das Granulat **dadurch gekennzeichnet ist, dass** es ferner mindestens ein Verdickungsmittel enthält, das aus den Gummis pflanzlichen, mikrobiellen und/oder synthetischen Ursprungs ausgewählt ist, wobei die Gesamtmenge des oder der Verdickungsmittel in dem Granulat zwischen 1 und 4 Gew.-%, bezogen auf das Gesamtgewicht des Granulats, beträgt.

16. Granulat nach Anspruch 15, enthaltend 1 bis 50 Gew.-%, bezogen auf das Gesamtgewicht des Granulats, anionischer Tenside.

## Claims

1. Cosmetic composition in dry form, suitable for being topically applied to the skin and/or keratin fibres, comprising at least 50% by volume of disintegratable granules having a size, measured by screening, between 150 and 2000 µm and containing a mixture of at least one anionic surfactant and at least one absorbent agent chosen from starches, said cosmetic composition being **characterised in that** said disintegratable granules further comprise at least one thickening agent chosen from gums of plant, microbial and/or synthetic origin, and the total amount of said thickening agent(s) in said composition is between 1 and 4% by weight relative to the total weight of the composition.

2. Cosmetic composition according to claim 1, comprising at least 75% by volume of said granules.

3. Cosmetic composition according to claim 1 or 2, wherein the size of said granules is between 315 and 1250 µm.

4. Cosmetic composition according to any one of claims 1 to 4, wherein the total amount of said thickening agent(s) chosen from gums of plant, microbial and/or synthetic origin, present in said disintegratable granules, is between 2 and 4% by weight relative to the total weight of the composition.

5. Cosmetic composition according to any one of claims 1 to 4, containing an amount between 1 and 50% by weight, relative to the total weight of the composition, of anionic surfactant(s).

6. Cosmetic composition according to any one of claims 1 to 5, **characterised in that** said anionic surfactant is chosen from isethionic acid derivatives and salts thereof.

7. Cosmetic composition according to any one of claims 1 to 6, **characterised in that** said granules further comprise at least one clay.

8. Cosmetic composition according to any one of claims 1 to 7, **characterised in that** it further comprises, outside said granules, at least one exfoliating agent.

9. Cosmetic composition according to any one of claims 1 to 8, wherein said granules contain an amount of polyol of less than 2% by weight, preferably of less than 1% by weight, relative to the total weight of the granules.

10. Cosmetic composition according to any one of claims 1 to 9, comprising a cosmetically active agent.

11. Cosmetic composition according to any one of claims 1 to 10, **characterised in that** it is packaged in single-dose form.

12. Method for washing and/or non-therapeutically cosmetically treating keratin fibres, comprising a step of applying, to said damp keratin fibres, a cosmetic composition as defined in any one of claims 1 to 11, optionally followed by massaging to develop foam, followed by an optional application time of 0 to 5 minutes, followed by rinsing of said keratin fibres with water.

13. Method for washing and/or non-therapeutically cosmetically treating the skin, comprising a step of applying, to said damp skin, a cosmetic composition as defined in any one of claims 1 to 11, optionally followed by massaging to develop foam, followed by an optional application time of 0 to 5 minutes, followed by rinsing of said skin with water.

14. Non-therapeutic cosmetic use, for exfoliating the skin of an individual's face and/or body, of a cosmetic composition as defined in any one of claims 1 to 11.

15. Disintegratable granule suitable for being topically applied to an individual's skin and/or keratin fibres, having a size, measured by screening, between 150 and 2000 µm and a containing a mixture of at least one anionic surfactant and at least one absorbent agent chosen from starches, said granule being **characterised in that** it contains at least one thickening agent chosen from gums of plant, microbial and/or synthetic origin, the total amount of said thickening agent(s) in said granule being between 1 and 4% by weight relative to the total weight of the granule.

16. Granule according to claim 15, containing 1 to 50% by weight, relative to the total weight of the granule, of anionic surfactant(s).
